Europäisches Patentamt

European Patent Office

Office européen des brevets

(11) Numéro de publication: **0 303 597 B1**

(12) **FASCICULE DE BREVET EUROPEEN**

(45) Date de publication de fascicule du brevet: **09.06.93** (51) Int. Cl.⁵: **A61K 31/195**

(21) Numéro de dépôt: **87901509.7**

(22) Date de dépôt: **04.03.87**

(86) Numéro de dépôt internationale :
**PCT/FR87/00052**

(87) Numéro de publication internationale :
**WO 88/06446 (07.09.88 88/20)**

Le dossier contient des informations techniques
présentées postérieurement au dépôt de la
demande et ne figurant pas dans le présent
fascicule.

(54) **Utilisation d'acide p-aminobenzöique pour la fabrication d'un médicament pour le traitement de photoallergies.**

(43) Date de publication de la demande:
**22.02.89 Bulletin 89/08**

(45) Mention de la délivrance du brevet:
**09.06.93 Bulletin 93/23**

(84) Etats contractants désignés:
**AT BE CH DE FR GB IT LI LU NL SE**

(56) Documents cités:
**FR-A- 2 591 482**

**Journal of the American Academy of Dermatology, vol. 7, No. 3, September 1982, Am.
Acad. Dermatol., M.A. Pathak
:"Sunscreens:Topical and systemic approaches for protection of human skin against
harmful effects of solar radiation", pp
285-312**

(73) Titulaire: **LABORATOIRES GALLIER
40, rue Lecuyer
F-93300 Aubervilliers(FR)**

(72) Inventeur: **CHEROUI, Jean
6, boulevard Suchet
F-75016 Paris(FR)**
Inventeur: **DJIANE, Alain
17, rue de Chartres
F-92200 Neuilly-sur-Seine(FR)**

(74) Mandataire: **Burtin, Jean-François
Cabinet GEFIB, 59, rue Edouard-Vaillant
F-92300 Levallois-Perret (FR)**

Rank Xerox (UK) Business Services
(3.10/3.6/3.3.1)

EP 0 303 597 B1

Australian Family Physician, vol. 6, No. 2, February 1977, M. Lane Brown: "Sense in the sun", pp 87-96

Dermatologic Clinica, vol. 4, No.2, April 1986, M.A. Pathak :"Sunscreens: Topical and systemic approaches for the prevention of acute and chronic sun-induced skin reactions", pp. 321-334

Farmaco, Edizione Pratica, vol. 36, No. 8, August 1981, A. Arancibia et al.: "Pharmacokinetic study of the percutaneous absorption of p-aminobenzoic acid from three sunscreen preparations", pp. 357-365

Chemical Abstracts, vol. 104, No. 6, 10 February 1986, (Columbus, Ohio, US), see page 366, abstract 39527t & JP-A-60174710

Manuila et coll.:"Dictionnaire Francais de Médecine et de Biologie",pages 302, 677 et 1038

## Description

La présente invention a pour objet l'utilisation de compositions pharmaceutiques à base d'acide p-aminobenzoïque ou d'un de ses sels pour la fabrication d'un médicament oral pour le traitement des lucites allergiques estivales.

Elle a plus particulièrement pour object l'utilisation de compositions pharmaceutiques caractérisées en ce qu'elles renferment comme principe actif une quantité photoprotectrice d'acide p-aminobenzoïque (PABA) ou d'un de ses sels avec une base minérale ou organique en mélange ou en association avec un ou plusieurs excipients inertes non-toxiques, pharmaceutiquement-acceptables, destinés à la voie orale.

Pour cet usage nouveau l'acide p-aminobenzoïque ou un de ses sels est présenté sous une des formes qui conviennent pour l'administration par voie orale par absorption digestive comme par exemple les comprimés nus ou enrobés par un enrobage entérique, les comprimés à plusieurs noyaux, les dragées, les gélules, les capsules, les pilules, les granulés, les poudres aromatisées ou non, les cachets, les sachets, les poudres lyophilisées ainsi que les formes à libération retardée.

La quantité photoprotectrice d'acide p-aminobenzoïque s'échelonne entre 400 et 1 000 mg par prise unitaire, La posologie journalière s'échelonne entre 1,5 g et 6 g chez l'adulte.

L'action photoprotectrice de l'acide p-aminobenzoïque en application locale a déjà été mise en évidence par ROTHMAN et confirmée par LANGNER & KLINGMAN. Cette action entraine un bronzage, donc une pigmentation de la peau sous l'influence de la lumière, plus intense et plus durable. L'étude histologique de la peau montre une augmentation du pigment mélanique. Alors que chez les sujets non traités le pigment mélanique est réparti irrégulièrement avec une tendance à se localiser dans la région supra nucléaire, on observe chez les sujets traités une augmentation des granules pigmentaires finement dispersés dans le cytoplasme et une forte augmentation de la concentration en mélamine.

Toutes les études thérapeutiques avaient donc précédemment essentiellement été, effectuées par voie externe en appliquant à titre préventif des crèmes à base d'acide p-aminobenzoïque sur les parties exposées de la peau.

En outre, les études cliniques effectuées par voie générale ont déjà mis en évidence un effet antidyspnéique, notamment par voie intra-veineuse, un effet de repigmentation utilisé dans le traitement du vitiligo et de la canitie. Le PABA est considéré comme n'ayant pas ou peu d'activité de protection contre d'érythème solaire lorsqu'il est administré oralement.

La présente invention a pour objet l'utilisation de PABA dans de compositions. thérapeutiques destinées à prévenir ou à traiter des phénomènes photo-toxiques dûs à une photosensibilisation et dénommés lucites, notamment les lucites estivales bénignes.

Cette affection se traduit par des éruptions simples ou prurigineuses ou des phénomènes urticarines plus particulièrement chez les sujets à peau claire. Cette maladie est indépendant des troubles du bronzage ou des érythèmes solaires déclanchés par une exposition au soleil dès le premier jour d'une période de vacances ou de voyage dans un pays ensoleillé.

Les compositions pharmaceutiques utilisées selon l'invention permettent un traitement préventif ou curatif des lucites estivales bénignes et leur effet est amélioré lorsque le traitement est commencé 15 jours avant l'exposition au soleil pendant des vacances. Le traitement est poursuivi pendant une durée totale de 3 à 4 semaines.

L'effet des compositions pharmaceutiques selon l'invention, attesté par de nombreuses études cliniques, parait nouveau et surprenant car des études effectuées aux Etats-Unis par WILLIS & KLINGMAN (Arch. Dermato/ 102 (1970) 416) avaient permis de conclure :

"Actuellement aucun médicament n'est connu qui puisse assurer une protection solaire par administration orale ou parentérale...

Cinq sujets ont reçu 12 g d'acide p-aminobenzoïque chaque jour en trois fractions pendant 10 jours. Le PI immédiat pour chaque sujet a été déterminé avant et après le dernier jour d'administration du médicament.

En aucun cas le PI n'a été augmenté après ingestion d'acide p-aminobenzoïque. Vraisemblablement la quantité qui atteint l'épiderme et le derme est soit modifiée soit trop faible pour jouer un rôle important"

En d'autres termes, ces auteurs, malgré une administration à dose élevée par voie digestive, avaient été incapables de mettre en évidence un effet photoprotecteur et en avaient donc conclu que l'acide p-aminobenzoïque n'avait pas d'effet photoprotecteur.

Les lucites sont les plus fréquentes des photodermatoses idiopathiques. Bien que ces affections restent encore difficiles à définir sur le plan nosologique, deux catégories peuvent être différenciées :

- la lucite estivale bénigne

- la lucite polymorphe correspondant à un cadre anatomo-clinique comprenant différentes variétés morphologiques de photodermatoses.

La lucite estivale bénigne a reçu sa dénomination en raison de sa survenue en été et de son évolution aiguë et bénigne.

Elle atteint principalement les femmes. L'éruption apparait en quelques heures ou quelques jours, après une exposition intense et prolongée au soleil, surtout pendant les vacances d'été.

La topographie des lésions est assez évocatrice par la prédominance au niveau de décolleté, des épaules, membres supérieurs et parfois des membres inférieurs. La face est généralement respectée.

L'éruption est très prurigineuse.

Elle se présente sous la forme d'un semis de petites papules érythémateuses s'égrégeant de façon assez dense, donnant une sensation de granité, d'érythème papulo-vésiculeux ou de placards oedémateux.

L'évolution est également très évocatrice : en effet, malgré la répétition des expositions, l'éruption s'étténue généralement en une dizaine de jours pour s'effacer à la fin de la 2ème semaine dès que le sujet a acquis une pigmentation suffisante.

Elle ne laisse pas de cicatrices, parfois une discrète hypochromie transitoire. Cette amélioration au cours de l'été constitue un bon élément clinique d'orientation, néanmoins la L.E..B peut persister pendant toute la durée des vacances estivales.

Au cours des vacances suivantes, cette lucite va réapparaitre lors d'une exposition solaire, intense et brutale. Cette récidive affecte souvent la même topographie et parfois même de nouveaux territoires. Mais dès l'installation du bronzage, les lésions disparaissent.

Les compositions pharmaceutiques utilisées selon l'invention, bien que la dose de principe actif utilisé soit sensiblement plus faible, assurent certainement une meilleure absorption de celui-ci et une distribution plus importante dans tout l'organisme.

Lorsque le principe actif est un sel de l'acide p-aminobenzoïque, il peut être un sel de métal alcalin comme le sel de sodium, de potassium, de lithium, d'ammonium, de cesium ou de rubidium ; un sel de métal alcalinoterreux comme un sel de calcium ou de strontium ; un sel de magnesium, un sel de zirconium, un sel d'aluminium, un sel de métal ferreux comme un sel de fer ou de manganèse ; un sel de terre rare comme un sel de lanthane, de scandium ou de germanium ; un sel de base organique comme une sel d'amine aliphatique, d'aminoalcool, d'aryl alcoylamine comme un sel de benzylamine, un sel d'aminosucre comme la glucosamine ou la méthylglucamine, un sel d'amino acide basique comme la lysine, l'arginine ou la citrulline, un sel de base guanidinique comme l'agmatine, la créatinine, la créatine, ou la streptamine ; un sel de peptide comme la clupeine, la spermine, la spermidine ou la salmine.

Ces sels peuvent être solubles dans les milieux aqueux ou insolubles dans ce milieu. Leur choix dépend essentiellement de la préférence du thérapeute ou de la commodité de leur incorporation dans la forme galénique désirée.

Parmi les excipients utilisés dans la réalisation des compositions pharmaceutiques selon l'invention, on citera les amidons de blé ou de maïs, le lactose, les celluloses, les alcoyl celluloses, les phosphates ou carbonates de métaux alcalino-terreux, le carbonate de magnesium, le phosphate de magnesium, les produits édulcorants comme la saccharose, les saccharrinates ou l'aspartam, des produits colorants comme le rouge coccine ou le jaune d'alizarine.

L'invention comprend également un procédé d'obtention des compositions pharmaceutiques agissant sur les leucites estivales allergiques caractérisé en ce qu'on incorpore ou mélange l'acide p-aminobenzoïque ou un de ses sels d'addition avec une base minérale ou organique en quantité photoprotectrice avec un ou plusieurs excipients inertes, non-toxiques, pharmaceutiquement-acceptables, adaptés pour l'administration par voie orale.

Les exemples suivants illustrent l'invention sans toutefois la limiter.

EXEMPLE I

| Sachets d'acide p-aminobenzoïque | |
|---|---|
| Acide p-aminobenzoïque (chlorhydrate) | 1 g |
| Amidon de maïs | 0,20 g |
| Stéarate de magnesium | 0,02 g |
| Carbonate de calcium | 0,40 g |
| Saccharose | 1 g |
| Lauryl sulfate de sodium pour un sachet fini à 2,50 g | 0,03 g |

EXEMPLE II

| Comprimés effervescents à base d'acide p-aminobenzoïque | |
|---|---|
| Acide p-aminobenzoïque | 1 kg |
| Carbonate de calcium | 0,150 kg |
| Bicarbonate de sodium | 0,600 kg |
| Amidon de maïs | 0,080 kg |
| Saccharose | 0,150 kg |
| Talc | 0,050 kg |
| Lactose pour mille comprimés terminés à 2,85 g | 0,470 kg |

EXEMPLE III

| Sachets d'acide p-aminobenzoïque (sel de lysine) | |
|---|---|
| Acide p-aminobenzoïque | 0,70 g |
| Lysine base | 0,90 g |
| Lactose | 0,25 g |
| Polymère d'éthylène glycol et de propylène glycol commercialisé sous la marque PLURONIC F 68 | 0,10 g |
| Sucre glace pour un sachet | 0,80 g |

EXEMPLE IV

| Granulés à base de p-aminobenzoate de calcium | |
|---|---|
| p-aminobenzoate de calcium | 4,75 g |
| Phosphate tricalcique | 27 g |
| Carbonate de calcium | 14,50 g |
| Ethyl callulose Hydroxypropylmethylcellulose | 0,90 g |
| Sucre glace | |
| Sirop de sucre q.s. | 25,75 g |

EP 0 303 597 B1

EXEMPLE V

Utilisation des compositions pharmaceutiques à base d'acide p-aminobenzoïque dans le traitement des lucites estivales bénignes.

L'étude de l'acide p-aminobenzoïque en comprimés sur les lucites estivales bénignes a été effectuée de façon multicentrique.

La difficulté principale réside dans la définition de cette affection dont le cadre nosologique reste assez mal déterminé. La lucite estivale bénigne est une affection qui atteint principalement les femmes.

L'éruption apparait après quelques heures ou les premiers jours, à la suite d'une exposition au soleil pendant les vacances d'été. La topographie des lésions est assez évocatrice avec une atteinte du haut du tronc, notamment du décolleté, des épaules et des membres supérieurs et parfois des membres inférieurs. La face est généralement respectée. L'éruption est très prurigineuse. Elle est constituée d'un semis de petites papules érythémateuses en plaques, donnant une sensation de granité, ou de lésions papulo-vésiculeuses, ou de placards oedémateux.

L'évolution est également très évocatrice : l'affection s'atténue progressivement pour disparaitre en 8 à 15 jours après l'installation du bronzage.

On a proposé à ces patients de prendre 2 comprimés matin et soir d'acide p-aminobenzoïque à 0,500 g en débutant 8 jours avant leur départ en vacances et pendant une durée totale de 3 ou 4 semaines.

La population étudiée est de 89 patients au total dont 72 femmes et 17 hommes.

Le diagnostic de lucite estivale bénigne ne posait pas de problèmes dans la majorité des cas.

Il s'agissait dans la plupart des cas d'une affection qui durait depuis plusieurs années.

Les résultats obtenus sont exposés en tenant compte d'une part, des résultats globaux observés avec les comprimés à base d'acide p-aminobenzoïque à 0,50 % d'autre part, en comparant chez 23 des patients les résultats obtenus par les comprimés selon l'invention à ceux observés antérieurement avec d'autres produits.

RESULTATS GLOBAUX

On a classé les résultats en trois groupes :
- très bons résultats pour les patients n'ayant eu aucune éruption ou signe fonctionnel pendant leurs vacances.
- bons résultats pour les patients ayant été nettement améliorés par rapport aux années précédentes.
- résultats nuls pour les patients chez lesquels le traitement n'a eu aucune efficacité.

Les résultats qui ont été obtenus sur les 89 patients testés s'établissent comme il est indiqué dans le tableau III :

| Résultats (sur 89 patients) | Nombre de patients | Pourcentage |
|---|---|---|
| Très bons résultats | 37 | ) ( 80% ) |
| Bons résultats | 34 | |
| Résultats nuls | 18 | 20% |

et se répartissent donc en 80 % de résultats globaux positifs contre 20 % de résultats nuls.

Parmi les bons résultats (34 cas) on s'est efforcé de faire préciser aux patients les caractéristiques de leur éruption.

Dans la plupart des cas il s'agit d'une éruption prurigineuse, moins intense que les autres années, mais on a aussi observé des modifications des caractères cliniques de leur lucite par rapport aux antécédents :
- éruption sans prurit dans 2 cas
- éruption retardée du 8è au 10ème jour dans 4 cas
- éruption limitée à une région peu étendue
- éruption sur une région qui était habituellement épargnée notamment au visage dans 1 cas

6

- phénomènes urticariens mineurs dans 4 cas.

Les améliorations même partielles sont tout de même considérées par les patients comme globalement positives.

Parmi les résultats nuls, on n'a noté qu'une seule aggravation après quelques jours d'exposition au soleil avec "photosensibilisation". On ne peut donc pas dire s'il s'agit d'une allergie vraie ou d'une photo-allergie.

Comparaison de l'action des compositions selon l'invention et celles des traitements antérieurs.

Seules 23 observations sur 89 font mention des traitements antérieurs. Dans tous les cas observés, le traitement par l'acide p-aminobenzoïque s'est révélé efficace par rapport aux autres médications (Cf. tableau IV).

TABLEAU IV

| Améliorations dues à l'acide p-aminobenzoïque | par rapport |
|---|---|
| 8 | Nicrobion [r] |
| 7 | Antihistaminiques avec ou sans Nicobion [r] |
| 7 | Y globulines, antihistaminiques Nicobion, antipaludéen de synthèse |
| 1 | Phenoro [r] |
| 1 | Celestamine [r] |

Résultats selon les différents paramètres

Enfin, il a paru intéressant de déterminer le rôle que pouvaient jouer un certain nombre de paramètres tels que le phototype, le sexe, l'âge et l'ancienneté de la maladie.

On a constaté que les résultats n'étaient influencés :

- ni par le phototype des patients. Cependant, pour les phototypes clairs, il y aurait peut-être des résultats plus favorables.
- ni par le sexe : 28 très bons résultats sur 72 cas féminins et quelques très bons résultats sur 17 cas masculins.
- ni par l'âge, ni par l'ancienneté de la maladie (quant ce paramètre était connu).

Résultats sur le bronzage

A la suite des travaux de LANGNER & KLIGMAN, montrant un accroissement de l'activité de la mélanogénèse après application locale de PABA en solution alcoolique et exposition aux rayons ultraviolets, il a paru intéressant de savoir si les sujets traités pour une lucite estivale bénigne avaient eu une pigmentation solaire plus importante que les autres années.

Chez la plupart des sujets, le bronzage a été nettement amélioré par rapport aux années précédentes. On peut donc dire qu'il y a certainement eu accroissement de l'activité de la mélanogénèse.

Le test de SAIDMAN classiquement utilisé pour déterminer l'effet photoprotecteur de crèmes solaires a montre que l'acide p-aminobenzoïque à 500 mg possède un effet photoprotecteur par voie systémique en protégeant contre les ultraviolets B.

**Revendications**

1.  Utilisation de l'acide p-aminobenzoïque ou d'un de ses sels d'addition avec une base minérale ou organique en vue de la réalisation d'un médicament actif, par voie orale, pour le traitement des lucites allergiques estivales.

2.  Utilisation selon la revendication 1. dans laquelle le principe actif est l'acide p-aminobenzoïque.

3.  Utilisation selon la revendication 1. dans laquelle le principe actif est le p-aminobenzoate de Calcium.

**4.** Utilisation selon la revendication 1. dans laquelle le principe actif est le p-aminobenzoate de Lysine.

**5.** Utilisation selon les revendications 1 à 4. dans laquelle le médicament actif pour le traitement des lucites allergiques estivales renferme de 400 à 1000 mg d'acide p-aminobenzoïque.

**6.** Procédé pour la réalisation d'un médicament actif, par voie orale, pour le traitement des lucites estivales allergiques dans lequel on incorpore ou on mélange une quantité efficace d'acide p-aminobenzoïque ou d'un de ses sels avec une base minérale ou organique, à un ou plusieurs excipients ou véhicules inertes, non-toxiques, pharmaceutiquement acceptables, adaptés à l'administration par voie orale.

**Claims**

**1.** Use of p.aminobenzoic acid or one of its addition salt with a mineral or organic base intended for the realization of an orally-active medicine for the treatment of allergic summer lucitis.

**2.** Use according to claim 1° wherein the active ingredient is p.aminobenzoic acid.

**3.** Use according to claim 1° wherein the active ingredient is Calcium p.aminobenzoate.

**4.** Use according to claim 1° wherein the active ingredient is Lysine p.aminobenzoate.

**5.** Use according to claims 1 to 4° wherein the drug active for the treatment of allergic summer lucitis contains from 400 to 1000 mg of p.aminobenzoic acid.

**6.** Process for the realization of a drug, active per oral way, for the treatment of allergic summer lucitis, in which one admixes or incorporates an efficacious amount of p.aminobenzoic acid or one of its salts with a mineral or organic base, to one or several inert, non-toxic, pharmaceutically-acceptable carriers or vehicles, intended for administration by oral way.

**Patentansprüche**

**1.** Verwendung von p.Aminobenzoesäure oder einen seiner Additionsalze mit einer anorganischen oder organischen Base, in Hinsicht auf die Verwirklichung eines durch oral Abreichung wirksamen Arzneimittels fur die Behandlung der allergischen sommerlichen Luziten.

**2.** Verwendung nach Anspruch 1°, worin der Wirkstoff p.Aminobenzoesäure ist.

**3.** Verwendung nach Anspruch 1°, worin der Wirkstoff Calzium p.Aminobenzoat ist.

**4.** Verwendung nach Anspruch 1°, worin der Wirkstoff Lysin p.Aminobenzoat ist.

**5.** Verwendung nach Anspruch 1°, worin das für die Behandlung von allergischen sommerlichen Luziten, wirksames Arzneimittel, von 400 bis 1000 mg p.Aminobenzoesäure enthält.

**6.** Verfahren fur die Verwirklichung einer durch oralen Abreichung fur die Behandlung von allergischen sommerlichen Luziten wirksamen Arzneimittels, worin man eine wirksame Menge an p.Aminobenzoesäure oder eine seiner Salze mit einer organischen oder anorganischen Base zu eine oder mehrere ungiftigen, inerten, pharmazeutischverträliche, für Abreichung durch oralen Weg geeigne-te Trägern or Vehikeln.